## Europäisches Patentamt

## European Patent Office

## Office européen des brevets

(11) Veröffentlichungsnummer: **0 159 599**
**B1**

(12) # EUROPÄISCHE PATENTSCHRIFT

(45) Veröffentlichungstag der Patentschrift:
02.08.89

(21) Anmeldenummer: **85104129.3**

(22) Anmeldetag: **04.04.85**

(51) Int. Cl.⁴: **C 07 C 67/31,** C 07 C 69/708, C 07 C 121/407 // C07C67/343

(54) Verfahren zur Herstellung von Alkoxymethylenverbindungen von Essigestern und substituierten Essigestern.

(30) Priorität: **26.04.84 DE 3415475**

(43) Veröffentlichungstag der Anmeldung:
**30.10.85 Patentblatt 85/44**

(45) Bekanntmachung des Hinweises auf die Patenterteilung:
**02.08.89 Patentblatt 89/31**

(84) Benannte Vertragsstaaten:
**AT BE CH DE FR GB IT LI LU NL SE**

(56) Entgegenhaltungen:
**EP-A-0 001 740**
**EP-A-0 090 185**

(73) Patentinhaber: **HÜLS AKTIENGESELLSCHAFT, Paul-Baumann- Strasse 1, D-4370 Marl 1 (DE)**

(72) Erfinder: **Englaender, Fritz, Dr., Flossweg 10, D-5300 Bonn 2 (DE)**
Erfinder: **Vogt, Wilhelm, Dr., Kleiststrasse 39, D-5000 Köln 40 (DE)**

LIBER, STOCKHOLM 1989

## Beschreibung

Die Erfindung betrifft besonders ein verbessertes Verfahren zur Herstellung von Methoxymethylenverbindungen von Essigsäuremethylester, der gegebenenfalls substituiert sein kann, sowie von Ethoxymethylenverbindungen von unsubstituierten und substituierten Essigsäureethylestern. Insbesondere werden nach dem Verfahren Methoxymethylenverbindungen von Malonsäuredimethylester, weiterhin von Cyanessigsäuremethylester, Methoxyessigsäuremethylester und Essigsäuremethylester hergestellt.

Entsprechend sind Ethoxymethylenverbindungen besonders von Malonsäurediethylester, weiterhin von Cyanessig-, Methoxyessig-, Ethoxyessig- und Essigsäureethylestern herstellbar. Bei Bedarf sind auch Alkoxymethylenverbindungen mit gesättigten aliphatischen Alkoxygruppen von 1 bis 6 C-Atomen der genannten Ester herstellbar, die in den Estergruppen sehr bevorzugt dieselben Alkylreste mit 1 bis 6 C-Atomen wie in der Alkoxymethylengruppe besitzen. Die Alkylreste können geradkettig oder verzweigt sein.

Ein besonderer Vorteil des Verfahrens besteht darin, daß Enolether von Hydroxymethylenverbindungen, die nur als Na-Salze beständig sind, wie die Na-Salze von Hydroxymethylenessigsäuremethylester und Hydroxymethylenmethoxyessigsäuremethylester, hergestellt werden können.

Es ist bekannt, die Methoxymethylenverbindung aus Malonsäuredimethylester mit Trimethylorthoformiat in Gegenwart von Acetanhydrid und ZnCl$_2$ umzusetzen (Claisen, A. 297 1 (1897), DE-AS 24 26 964). Nachteilig an dem Verfahren ist jedoch, daß es lange Reaktionszeiten und einen hohen Destillationsaufwand erfordert, wenn gute Ausbeuten erzielt werden sollen.

Weiterhin wird nach der DE-OS 18 00 352 Malonsäuredimethylester mit CO in Gegenwart von Natriummethylat und Methanol zum Na-Salz des Hydroxymethylenmalonsäuredimethylesters umgesetzt und dieses in einem inerten Lösemittel wie Toluol mit Dimethylsulfat in Methoxymethylenmalonsäuredimethylester überführt. Außer der großen Toxizität des Dimethylsulfats hat die Reaktion noch den Nachteil, daß nur eine Methylgruppe des Dimethylsulfats ausgenutzt wird. Alkohole anstelle von Dialkylsulfat führen nicht zu Alkoxymethylenverbindungen.

Es bestand daher die Aufgabe, Alkoxymethylenverbindungen, besonders Methoxymethylenverbindungen, von Essigsäureestern und substituierten Essigsäureestern mit 1 bis 6 C-Atomen im Alkylrest der Estergruppe, besonders von Methylestern, in einfacher Weise und in hoher Ausbeute herzustellen.

Als substituierte Essigsäureester sind dabei besonders Cyanessigsäureester, Alkoxyessigsäureester mit einer Alkoxygruppe mit geradkettigen oder verzweigten Alkylresten von 1 bis 6 C-Atomen, Phenyl- oder Naphthylessigsäureester oder solche mit den Substituenten -COOR, worin R die definierten Alkylreste sind, welche Stoffe Malonsäuredialkylester sind.

Es wurde gefunden, daß man Alkoxymethylenverbindungen, besonders Methoxymethylenverbindungen, von Essiesäureestern und substituierten Essigsäureestern in einfacher Weise und hoher Ausbeute dadurch herstellen kann, daß man die jeweiligen Hydroxymethylenessigsäurealkylester, besonders Hydroxymethylenessigsäuremethylester, und/oder deren Alkalisalze mit überschüssigem Alkohol in Gegenwart von HCl und einem wasserbindenden Mittel umsetzt.

Wasserbindende Mittel sind an sich bekannt, z. B. in Form von Schwefelsäure bei der Reaktion nach EP-A-0 001 740. Dabei wird ausgenutzt, daß wäßrige Schwefelsäure nicht flüchtig ist und zugleich Wasser bindet. Diese Maßnahme und Wirkung ist jedoch gänzlich verschieden von der vorliegenden Erfindung.

Gegenstand der Erfindung ist daher ein Verfahren zur Herstellung von Alkoxymethylenverbindungen, besonders von Methoxymethylenverbindungen, von substituierten oder unsubstituierten Essigsäurealkylestern, besonders Essigsäuremethylestern, dadurch gekennzeichnet, daß man die entsprechende Hydroxymethylenverbindung der substituierten oder unsubstituierten Essigsäurealkylester bzw. deren Alkalisalz mit überschüssigem Alkohol, bevorzugt Methanol und a) dem aus einem Alkylnitril, vorzugsweise Acetonitril, Methanol und HCl entstehende salzsaure Iminoester und/oder b) mit Kieselsäuretetraalkylester, besonders Tetramethoxysilan in Gegenwart von HCl und/oder c) mit einem Gemisch aus SiCl$_4$ und Alkanol, bevorzugt Methanol als wasserbindendem Mittel, umsetzt.

Von besonderem Wert ist das Verfahren für die Herstellung von Methoxymethylenmalonsäuredimethylester. Daneben kann das Verfahren zur technischen Herstellung von Ethoxymethylenmalonsäurediethylester sowie der Methoxymethylenverbindungen von Cyanessigsäuremethylester, Methoxyessigsäuremethylester, Essigsäuremethylester und schließlich zur Herstellung beliebiger Alkoxymethylenverbindungen dienen.

Das Verfahren wird mit überschüssigem Alkanol ausgeführt. Vorzugsweise dient das Alkanol als Lösungsmittel oder Suspendiermittel. Sehr bevorzugt sollen mindestens 1,1 Mol Alkanol je Mol Ausgangsstoff anwesend sein. Bevorzugtes Alkanol ist Methanol für die Herstellung von Methoxymethylenverbindungen. zur Herstellung von Ethoxymethylenverbindungen ist Ethanol, zur Herstellung weiterer Alkoxymethylenverbindungen das jeweilige Alkanol zu wählen. Zweckmäßig sollen die Alkylgruppe der Alkoxymethylengruppe und die Alkylgruppe im Esterrest gleich sein.

Als Ausgangsstoffe werden die Alkalisalze der jeweiligen Alkoxymethylenverbindungen, besonders die Natriumsalze, bevorzugt. Sehr bevorzugt wird das Verfahren anschließend an die Herstellung der Alkalisalze von Hydroxymethylenverbindungen durch Formylierung des Esters mit CO in Alkanol in Gegenwart von Alkalialkoholat ausgeführt. Das jeweilige Alkanol dient auch dort als Lösungsmittel. Besonders gilt das für die Reaktionslösung von Na-Hydroxymethylenmalonsäuredimethylester in Methanol. Die Formylierung kann auch mit Alkylformiat in Gegenwart von Alkalialkoholat erfolgen.

Bei dem Verfahren soll HCl im Überschuß vorhanden sein. HCl ist daher als Gas zuzugeben, soweit es nicht bei der Reaktion der wasserbindenden Mittel entsteht. Während der Reaktion soll die Konzentration des freien HCl 0,5 bis 10 Gew.-%, vorzugsweise 3,0 bis 6,0 Gew.-%, bezogen auf das Gewicht der Reaktanden im Reaktionsgemisch, betragen. Als wasserbindende Mittel eignen sich solche, die bei der Reaktion und Aufarbeitung nicht erneut Wasser in Freiheit setzen. Besonders eignen sich wasserbindende Mittel, die mit Wasser stabile Umsetzungsprodukte bilden, die bei der Reaktion und Aufarbeitung inert sind.

Folgende Ausführungsformen haben sich besonders bewährt. Zum einen kann bei der Herstellung von besonders Methoxymethylenverbindungen des substituierten bzw. unsubstituierten Essigsäuremethylesters, ganz besonders des Methoxymethylenmalonsäuredimethylesters, Methanol sowie HCl und als wasserbindendes Mittel ein aliphatisches Nitril mit 2 bis 6 C-Atomen, besonders Acetonitril, zugesetzt werden. Der sich aus Acetonitril, Methanol und HCl bildende Acetiminomethylester · HCl reagiert mit Wasser zu Methylacetat und NH₄Cl. Auf diese Weise wird das Wasser aus dem Gleichgewicht entfernt und die Ausbeute an Methoxymethylenmalonsäuredimethylester beträchtlich erhöht. Das Nitril soll in Mengen von 1,02 bis 2,0, vorzugsweise 1,05 bis 1,25 Mol, je Mol Ausgangsstoff eingesetzt werden.

Zweckmäßig dienen bei Verwendung von Alkylnitrilen die freien Hydroxymethylenverbindungen als Ausgangsstoffe. Zum anderen können Tetraalkoxysilane mit 2 bis 6 C-Atomen in der Alkylgruppe, sehr bevorzugt Tetramethoxysilan, weiterhin auch Tetraethoxysilan, als wasserbindendes Mittel zusammen mit HCl zugesetzt werden. Soweit verfügbar, kann auch SiCl₂ (OCH₃)₂ bzw. SiCl₃ (OCH₃) Verwendung finden. Bei der Reaktion mit dem Wasser entstehen hochmolekulare Siloxane, von denen der Enolether leicht abdestilliert werden kann.

Zum weiteren kann als wasserbindendes Mittel SiCl₄ zugesetzt werden. SiCl₄ reagiert mit Alkanol zu HCl und Tetraalkoxysilan bzw. kondensierten Silanen. Aus den Alkalisalzen setzt HCl die Hydroxymethylenverbindung in Freiheit. Bevorzugt sind daher als Ausgangsstoffe die Alkalisalze sowie weiterhin die Methylester.

Methanolische Lösungen sind bevorzugt. Ethanolische Lösungen sind zur Herstellung von Ethoxymethylenverbindungen Lösungen des jeweiligen Alkanols zur Herstellung der jeweiligen Alkoxymethylenverbindungen bevorzugt. Mit Vorteil kann direkt von der alkoholischen Suspension von Natriumhydroxymethylen-malonsäuredimethylester bzw. weiterer Alkalisalze von Hydroxymethylenessigsäuremethylester ausgegangen werden, wie sie bei der Formylierung von den genannten Essigsäurealkylestern, z. B. Dimethylmalonat in Gegenwart von Natriummethylat mit CO oder Alkylformiat, mit Alkyl in der genannten Bedeutung, ggf. in Anwesenheit der Alkylalkohole mit Alkyl in der gleichen Bedeutung, erhalten wird. HCl setzt aus dem Na-Salz die Hydroxymethylenverbindung in Freiheit. Auf diese Weise ergibt sich als zusätzlicher Vorteil, daß das Reaktionsprodukt der Formylierungsstufe nicht isoliert zu werden braucht. Der Hydroxymethylenessigsäuremethylester reagiert in Anwesenheit von SiCl₄ mit Alkanolen, die in der Formylierungsstufe ohnehin im Überschuß vorhanden sind, zu der Alkoxymethylenverbindung.

Das bei der Umsetzung von SiCl₄ und dem Alkanol gebildete HCl fungiert hierbei als Katalysator. Das bei der Umsetzung gebildete Wasser setzt sich mit Tetraalkoxysilan zu Polysiloxanen um und wird auf diese Weise aus dem Acetalisierungsgleichgewicht entfernt.

Die Menge an SiCl₄ bzw. Tetraalkoxysilan, die pro Mol gebildetes H₂O zuzusetzen ist, liegt zwischen 0,5 und 2,0 Mol. Bei Reaktion von 0,5 Mol entsteht theoretisch SiO₂, d.h. tatsächlich ein sehr hochmolekulares Siloxan mit nur noch wenigen Alkoxygruppen, bei Reaktion von 2,0 Mol entstehen Siloxane mit Siedepunkten über 200° C, überwiegend (CH₃O)₃SiOSi(OCH₃)₃. Sehr hochmolekulare Polysiloxane sind unerwünscht, weil sie Reaktionsprodukt bei der Destillation hartnäckig festhalten und nach beendeter Destillation schwer aus der Apparatur zu entfernen sind. Sehr niedermolekulare Polysiloxane gehen beim Abdestillieren des Reaktionsproduktes mit über und verunreinigen das Destillat. Die bevorzugte Menge an SiCl₄ liegt demnach zwischen 0,6 und 1,1 Mol SiCl₄ bzw. Tetraalkoxysilan pro Mol H₂O.

Die Umsetzung findet im allgemeinen bei Raumtemperatur statt. Es können jedoch auch erhöhte Temperaturen bis maximal 50° C angewendet werden. Niedrigere Temperaturen als 20° C bringen keinen Vorteil. Der bevorzugte Bereich liegt zwischen 20 und 40° C.

Die Reaktion kann im geschlossenen Gefäß bei Normaldruck ausgeführt werden.

Nach der Reaktion wird neutralisiert, gegebenenfalls vom entstandenen NaCl getrennt und z. B. durch Destillation aufgearbeitet.

Das von den Polysiloxanen abdestillierte Reaktionsprodukt enthält häufig noch Acetale d.h. Dialkoxymethylverbindungen, z. B. enthält Methoxymethylenmalonsäuredimethylester Anteile von Dimethoxymethylmalonsäuredimethylester. Durch Erhitzen auf 100 bis 200° C, vorzugsweise 140 bis 155° C, bevorzugt in Gegenwart von 0,1 bis 10 Gew.-%, vorzugsweise 0,9 bis 2,5 Gew.-%, eines sauren Katalysators wie z. B. KHSO₄, H₂SO₄ oder p-Toluolsulfonsäure wird Methanol abgespalten und man erhält in fast quantitativer Ausbeute Methoxymethylenmalonsäuredimethylester.

Die Methoxymethylenverbindungen sind wertvolle Zwischenprodukte zur Herstellung heterocyclischer Verbindungen. Beispielsweise wird Methoxymethylenmalonsäuredimethylester für Synthesen in der Chinolinreihe eingesetzt (vgl. Schofield K. u. J. C. E. Simpson: J. Chem. Soc. London 1946, 1033. Snyder H. R. J: Am. Chem. Soc. 68, 1204, 1251 (1946); 69, 371 (1947), Duffin, G.J. und J.D. Kendall: J. Chem. Soc. London 1948, 893).

**Beispiel 1**

160 g (1,0 Mol) Hydroxymethylenmalonsäuredimethylester werden in 288 g (9,0 Mol) Methanol gelöst und 50 g Chlorwasserstoffgas eingeleitet. Dann werden 55,2 g (1,35 Mol) Acetonitril zugegeben und 24 Std. bei Raumtemperatur stehen gelassen. Danach werden die Leichtsieder abdestilliert, der Rückstand mit Dichlormethan versetzt und $NH_4Cl$ abfiltriert. Das Filtrat wird mit 1,6 g $KHSO_4$ versetzt und unter Abdestillieren der flüchtigen Bestandteile bis 130°C erhitzt. Man erhält 138 g (0,79 Mol) Methoxymethylenmalonsäuredimethylester entsprechend 79,3 % der Theorie.

**Beispiel 2**

396 g (3,0 Mol) Dimethylmalonat 170,1 g Natriummethylat und 1425 g (44,5 Mol) Methanol werden bei 60°C und 45 bar CO-Druck reagieren lassen, bis keine CO-Aufnahme mehr stattfindet. In der Lösung entsteht das Na-Salz des Hydroxymethylenmalonsäuredimethylesters. Danach werden 382,5 g (2,25 Mol) Siliciumtetrachlorid bei einer Temperatur von 25° zugegeben. Zur Vervollständigung der Umsetzung wird noch 5 Std. bei 25°C gehalten. Durch Zugabe von Natriummethylat wird das Reaktionsgemisch auf pH 2 eingestellt und NaCl abfiltriert. Aus dem Filtrat gewinnt man durch Destillation unter Normaldruck Methylformiat und Methanol zurück, die wiederverwendet werden können. Durch Vakuumdestillation erhält man ein Gemisch von Methoxymethylenmalonsäuredimethylester und Dimethoxymethylmalonsäuredimethylester. Im Rückstand verbleiben die polymeren Kieselsäureester.

Das Destillät der Vakuumdestillation wird mit 2 g $KHSO_4$ versetzt und auf 150°C erhitzt. Nach Beendigung der Methanolabspaltung wird im Vakuum fraktioniert. Man erhält 433 g Methoxymethylenmalonsäuredimethylester entsprechend 82,3 % der Theorie bezogen auf eingesetztes Dimethylmalonat.

**Beispiel 3**

In einen Autoklav werden 690 g Toluol und 90,7 g Natrium-Methylat eingefüllt. Bei einer Temperatur von 50°C und einem CO-Druck von 50 bar wird ein Gemisch von 166,4 g Methoxyessigsäuremethylester und 355 g Methylformiat zudosiert. Sobald die CO-Aufnahme beendet ist, wird bei einer Temperatur von 25°C eine Mischung von 716 g Methanol und 130 g Siliciumtetrachlorid zugegeben. Nach Beendigung der Umsetzung wird mit Natriummethylat auf pH 2 gebracht und NaCl abfiltriert. Aus dem Filtrat werden die Leichtsieder Methylformiat, Methanol und Toluol unter Normaldruck abdestilliert. Das Reaktionsprodukt muß im Vakuum von den polymeren Kieselsäureestern abdestilliert werden. Es wird mit 2 g $KHSO_4$ versetzt und auf 150°C erhitzt. Nach Beendigung der Methanolabspaltung kann fraktioniert werden. Man erhält 177,5 g Methoxymethylenmethoxyessigsäuremethylester entsprechend 76 % der Theorie bezogen auf eingesetzten Methoxyessigsäuremethylester.

**Beispiel 4**

372 g Na-Salz des Hydroxymethylenessigsäuremethylesters werden in 1720 g Methanol suspendiert und bei 20°C mit 305 g Siliciumtetrachlorid versetzt. Das Reaktionsgemisch wird anschließend 12 Stunden stehen gelassen und danach mit Natriumethylat auf pH 2 gebracht und NaCl abfiltriert.

Nach dem Abdestillieren des Methanols wird der Rückstand im Vakuum von den Kieselsäureestern abdestilliert. Zu dem Destillat werden 2 g $KHSO_4$ gegeben und auf 130°C erhitzt. Sobald die Abspaltung des Methanols beendet ist, wird im Vakuum fraktioniert.

Man erhält 181 g Methoxymethylenessigsäuremethylester. Unter Berücksichtigung der Reinheit des Ausgangsproduktes (68-%-ig) entspricht dies einer Ausbeute von 76,5 % der Theorie.

**Beispiel 5**

164 g Na-Salz des Hydroxymethylencyanessigsäuremethylesters werden in 320 g Methanol suspendiert und bei 20°C mit 114 g Siliciumtetrachlorid versetzt. Nach 13 Stunden wird mit Natriummethylat ein pH von 2 eingestellt. Die weitere Aufarbeitung erfolgt wie in Beispiel 4.

Die Ausbeute beträgt 91 g Methoxymethylencyanessigsäuremethylester. Unter Berücksichtigung der Reinheit des Ausgangsproduktes (89-%-ig) entspricht dies einer Ausbeute von 65,9 % der Theorie.

**Beispiel 6**

160 g Diethylmalonat, 71,4 g Natriumethylat und 322 g Ethanol werden bei 60°C und 45 bar mit CO umgesetzt, bis keine weitere Aufnahme von CO stattfindet. Zu dem Reaktionsgemisch, mit dem entstandenen Na-methylenmalonsäurediethylester, wird bei einer Temperatur von 85°C 136 g Siliciumtetrachlorid gegeben und zur Vervollständigung der Umsetzung 5 Stunden auf 25°C gehalten. Durch Zugabe von Natriumethylat wird das Reaktionsgemisch auf pH 2 eingestellt und NaCl abfiltriert. Durch Destillation unter Normaldruck erhält man aus dem Filtrat Ethylformiat und Ethanol zurück, die wiederverwendet werden können. Aus dem Rückstand gewinnt man durch Vakuumdestillation ein Gemisch von Ethoxymethylenmalonsäurediethylester und Diethylmethylmalonsäurediethylester. Im Rückstand verbleiben die polymeren Kieselsäureester als bei 100°C ölige Flüssigkeit. Das Destillat der Vakuumdestillation wird mit 1,5 g KHSO$_4$ versetzt und auf 160°C erhitzt. Nach Beendigung der Abspaltung von Ethanol wird im Vakuum fraktioniert.

Als Vorlauf erhält man etwas Diethoxymethylmalonsäurediethylester, dessen Ethanol zusammen mit dem Acetal einer nachfolgenden Charge abgespalten werden kann.

Die Ausbeute an Ethoxymethylenmalonsäurediethylester beträgt 144,7 g entsprechend 67 % der Theorie bezogen auf eingesetztes Diethylmalonat.

**Patentansprüche**

1. Verfahren zur Herstellung von Alkoxymethylenverbindungen, besonders von Methoxymethylenverbindungen, von substituierten oder unsubstituierten Essigsäurealkylestern, besonders Essigsäuremethylestern, dadurch gekennzeichnet, daß man die entsprechende Hydroxymethylenverbindung der substituierten oder unsubstituierten Essigsäurealkylester bzw. deren Alkalisalz mit überschüssigem Alkohol, bevorzugt Methanol und a) dem aus einem Alkylnitril, vorzugsweise Acetonitril, Methanol und HCl entstehende salzsaure Iminoester und/oder b) mit Kieselsäuretetraalkylester, besonders Tetramethoxysilan in Gegenwart von HCl und/oder c) mit einem Gemisch aus SiCl$_4$ und Alkanol, bevorzugt Methanol als wasserbindendem Mittel, umsetzt.

2. Verfahren nach Anspruch 1, dadurch gekennzeichnet, daß das bei der Reaktion erhaltene Gemisch aus Enolether und Acetal thermisch und/oder in Gegenwart saurer Katalysatoren in reinem Enolether überführt wird.

3. Verfahren nach mindestens einem der Ansprüche 1 oder 2, dadurch gekennzeichnet, daß die Umsetzung bei Temperaturen von 20 bis 50°C, vorzugsweise bei 20 bis 40°C, durchgeführt wird.

4. Verfahren nach einem der Ansprüche 1 bis 3, dadurch gekennzeichnet, daß pro Mol gebildetes Wasser 0,5 bis 2,0 Mol SiCl$_4$ bzw. Si(OCH$_3$)$_4$, vorzugsweise 0,6 bis 1,1 Mol eingesetzt werden.

5. Verfahren nach einem der Ansprüche 1 bis 3, dadurch gekennzeichnet, daß pro Mol gebildetes Wasser 1,0 bis 2,0 Mol, vorzugsweise 1,05 bis 1,25 Mol Nitril eingesetzt wird.

6. Verfahren nach Anspruch 2, dadurch gekennzeichnet, daß die bei der Reaktion gebildeten Acetale bei 100 bis 200°C, vorzugsweise bei 140 bis 155°C, in die Enolether überführt werden.

7. Verfahren nach Anspruch 6, dadurch gekennzeichnet, daß die sauren Katalysatoren für die Methanolabspaltung in einer Menge von 0,1 bis 10 Gew.-%, vorzugsweise von 0,9 bis 2,5 Gew.-%, bezogen auf die Menge der Acetale, eingesetzt werden.

8. Verfahren nach mindestens einem der vorangehenden Ansprüche, dadurch gekennzeichnet, daß als Ausgangsstoff die Reaktionslösung aus der Herstellung der Alkalisalze der Hydroxymethylenverbindungen durch Formylierung dient.

**Claims**

1. Process for the production of alkoxymethylene compounds, specially of methoxymethylene compounds, from substituted or unsubstituted acetic acid alkyl esters, especially acetic acid methyl esters, characterised in that the corresponding hydroxymethylene compound of the substituted or unsubstituted acetic acid alkyl ester or alkali salt thereof is reacted with excess alcohol, preferably methanol a) with the imino ester acid salt originating from an alkylnitrile, preferably acetonitrile, methanol and HCl and/or b) with silicic acid tetraalkyl ester, especially tetramethoxy silane in the presence of HCl and/or c) with a mixture of SiCl$_4$ and alkanol, preferably methanol as water binding substance.

2. Process according to claim 1, characterised in that the mixture of enol ether and acetal obtained in the reaction is converted thermally and/or in the presence of acid catalysts into pure enol ether.

3. Process according to at least one of claims 1 or 2, characterised in that the reaction is carried out at temperatures of 20 to 50°C, preferably at 20 to 40°C.

4. Process according to one of claims 1 to 3, characterised in that 0.5 to 2.0 mol of SiCl$_4$ or Si(OCH$_1$)$_4$, preferably 0.6 to 1.1 mol are employed per mol of water formed.

5. Process according to one of claims 1 to 3, characterised in that 1.0 to 2.0 mol, preferably 1.05 to 1.25 mol of nitrile is employed per mol of water formed.

6. Process according to claim 2, characterised in that the acetals formed in the reaction are converted at 100 to 200°C, preferably at 140 to 155°C, into the enol ethers.

7. Process according to claim 6, characterised in that the acid catalysts for the splitting off of methanol are employed in an amount of 0.1 to 10 % by weight, preferably 0.9 to 2.5 % by weight, related to the amount of the acetals.

8. Process according to at least one of the foregoing claims, characterised in that the reaction solution from the production of the alkali salt of the hydroxymethylene compounds by formylation serves as starting material.

**Revendications**

1. Procédé de préparation de composés alcoxyméthyléniques, en particulier de composés méthoxyméthyléniques, d'esters alkyliques d'acide acétique substitué ou non substitué, en particulier des esters méthyliques de l'acide acétique, procédé caractérisé en ce qu'on fait réagir le composé hydroxyméthylénique correspondant de l'ester alkylique d'acide acétique, substitué ou non substitué, ou son sel alcalin avec un excès d'alcool, de préférence le méthanol, et a) l'iminoester chlorhydrique résultant d'un alkylonitrile, avantageusement l'acétonitrile, de méthanol et de HCl, et/ou b) avec un ester tétraalkylique d'acide silicique, en particulier le tétraméthoxysilane en présence de HCl et/ou c) avec un mélange de SiCl$_4$ et d'un alcanol, de préférence le méthanol, comme agent de fixation de l'eau.

2. Procédé selon la revendication 1, caractérisé en ce qu'on transforme thermiquement, et/ou en présence de catalyseurs acides, le mélange d'éther d'énol et d'acétal, obtenus dans la réaction, en un éther d'énol pur.

3. Procédé selon l'une au moins des revendications 1 ou 2, caractérisé en ce qu'on conduit la réaction à des températures de 20 à 50°C, avantageusement 20 à 40°C.

4. Procédé selon l'une des revendications 1 à 3, caractérisé en ce qu'on utilise, par mole d'eau formée, 0,5 à 2,0 moles de SiCl$_4$ ou de Si(OCH$_3$)$_4$, avantageusement 0,6 à 1,1 mole.

5. Procédé selon l'une des revendications 1 à 3, caractérisé en ce qu'on utilise, par mole d'eau formée, 1,0 à 2,0 moles, avantageusement 1,05 à 1,25 mole, de nitrile.

6. Procédé selon la revendication 2, caractérisé en ce qu'on transforme les acétals, formés dans la réaction, en opérant entre 100 et 200°C, avantageusement entre 140 et 155°C, en les éthers d'énol.

7. Procédé selon la revendication 6, caractérisé en ce qu'on utilise les catalyseurs acides pour la séparation du méthanol en une quantité de 0,1 à 10 % en poids, avantageusement de 0,9 à 2,5 % en poids, par rapport à la quantité des acétals.

8. Procédé selon l'une au moins des revendications précédentes, caractérisé en ce que la solution de réaction provenant de la préparation des sels alcalins des composés hydroxyméthyléniques, par formylation, sert de matière de départ (pour le présent procédé).